# EUROPEAN PATENT SPECIFICATION

(11) **EP 2 387 393 B1**
(45) Date of publication and mention of the grant of the patent: **16.11.2016**
(21) Application number: 10701586.9
(22) Date of filing: 13.01.2010
(51) Int. Cl.: A61K 9/20, A61K 9/70

(54) **UNIT ASSEMBLY FOR MULTIPLE FILM DOSAGES, APPARATUS, AND METHODS**
VERBUND MEHRER FILMDOSIERUNGSEINHEITEN, VORRICHTUNG UND HERSTELLUNG
ENSEMBLE UNITAIRE POUR DOSAGES DE FILM MULTIPLES, DISPOSITIF ET PROCÉDÉS

(30) Priority: 13.01.2009 US 144222 P
(43) Date of publication of application: 23.11.2011
(73) Proprietor: MonoSol Rx LLC, Warren, NJ 07059 (US)
(72) Inventor: HARIHARAN, Madhu, Greensboro NC 27410 (US); FUISZ, Richard, C., Beverly Hills, CA 90210 (US)
(74) Representative: ABG Patentes, S.L.
(86) International application number: PCT/US2010/020844
(87) International publication number: WO 2010/083183

(56) References cited:
- WO-A1-2009/052421
- WO-A2-2008/036299
- US-A1- 2003 224 044
- US-A1- 2006 147 493

## Description

### FIELD OF THE INVENTION

The present invention relates generally to asymmetrical film packaging systems and unit assemblies and processes to make same. Specifically, the present invention relates to packaging systems of multiple individual film strips, each film strip having an active within a polymer matrix, where the film strips are packaged within a unit assembly in an asymmetrical order to be readily distributable in single dose form to a user.

### BACKGROUND

Pharmaceuticals, biologicals, nutraceuticals, and therapeutic substances in general are typically required to be ingested in relatively precise amounts. Ingestible film strip technology allows for precise pharmaceutical amounts to be incorporated on or into a digestible film strip. Thus, a user requiring a dosage of a material would place an ingestible film strip in their mouth, to be consumed.

Ingestible film strips may be packaged individually, to prevent accidental ingestion of more than one film strip by a user. That is, a user may accidentally take more than one film strip together from a cassette style package, as the strips are very thin and the user may be unaware that they have removed multiple films at one time. However, individual packaging of each film strip has inefficiencies associated with it, as it significantly increases manufacturing costs. As the user has to unwrap each individual film strip package, there is a lead time associated with administering dosages and each film strip is susceptible to tearing and/or contamination by the user as the user attempts to open the film strip package. Additionally, certain users may find opening individual packages difficult, especially the elderly or those with arthritis or other such conditions.

WO 2008/036299 A2 discloses films that may be used to orally administer an active and which can be packaged on a backing and peeled off for use.

### SUMMARY OF THE INVENTION

An aspect of the present invention provides a unit assembly including: a plurality of individual film strips, each film strip having an active within a polymer matrix, the film strips having respective perimetrical edges, the plurality of individual film strips being alternatingly positioned next to one another such that a film strip perimetrical edge is at least partially offset from a subsequent film strip perimetrical edge, wherein the respective perimetrical edges of the film strips are directly adjacent to each other.

Another aspect of the present invention provides a method for making a unit assembly, the method including: providing at least two individual polymeric film strips, the film strips having respective perimetrical edges; and alternatingly overlaying the film strips onto one another such that the respective perimetrical edges of a first film strip is at least partially offset from the perimetrical edges of the each of the other film strips which are directly adjacent to a first film strip.

Still another aspect of the present invention provides a method for making a unit assembly, the method including: providing a sheet of an ingestible film, the film including a polymeric matrix having an active therein; cutting the sheet with at least a first cutter and a second cutter, the first cutter having a first orientation generally parallel to a longitudinal axis of the sheet, the second cutter having a second orientation generally perpendicular to the longitudinal axis of the sheet, wherein the first and second cutters create a plurality of individual film strips having substantially the same shape and size from the sheet; overlaying the individual film strips into a unit assembly such that each film strip does not completely overlay the perimeter of its adjacent film strips.

Still another aspect of the present invention provides an apparatus for making a unit assembly including: a mandrel on which a sheet of film is supported, the film including an active within a polymer matrix; at least two blades, a first blade having a first position relative to a sheet position and a second blade having an adjustable position relative to the sheet position wherein the second blade is rotatable.

Yet another aspect of the present invention provides a method of dosage retrieval, including: providing a unit assembly including a plurality of individual film strips packaged therein, each film strip having an active within a polymer matrix; removing a individual film strip from the unit assembly; and inspecting the individual film strip for a cue that the individual film is a single dosage.

These and other features of the invention will be more fully understood from the following description of specific embodiments of the invention taken together with the accompanying drawings.

### BRIEF DESCRIPTION OF THE DRAWINGS

Fig. 1 is a plan view of the asymmetrical film packaging system of the present invention showing the removal of single film strips from a unit assembly having two or more film strips.
Fig. 2 is a plan view of the unit assembly of Fig. 1 showing the removal of at least two individual film strips from the unit assembly.
Figs. 3A to 3D are plan views showing the formation of the L-shaped film strips and the overlaying thereof into the unit assemblies of Figs. 1 and 2.
Fig. 4 is a plan view of an alternative embodiment of one of the film strips of Fig. 1.
Fig. 5 is a plan view of at least two of the film strips of Fig. 4 in direct adjacent relation to one another.
Figs. 6A to 6D are plan views showing the formation of the trapezoidal-shaped film strips of Figs. 4 and 5.
Figs. 7A to 7C are plan views showing the formation of the triangle-shaped film strips.
Figs 8A through 8G depict various visual cues that may be employed with the unit assemblies of the present invention.
Fig. 9 depicts an embodiment of a method for making a unit assembly.
Fig. 10 depicts another embodiment of a method for making a unit assembly.
Fig. 11 depicts a flow chart of an apparatus for forming a unit assembly of the present invention.
Fig. 12 depicts an embodiment of a method of dosage retrieval.
Fig. 13A and 13B depict an alternate configuration of the film strip.

### DETAILED DESCRIPTION OF THE INVENTION

The embodiments of the present invention are directed to a unit assembly for dispensing individual ingestible films, an apparatus for making the individual film dosages, and methods of making and using the film dosages. The film dosages may be used to effectively and efficiently administer one or more agents to an individual in need thereof, for example various therapeutic agents including but not limited to pharmaceuticals, bioeffecting agents, medicaments, nutraceuticals, and/or cosmetic products. The individual film dosages may be desirably configured into an orientation with respect to one another so that an individual may remove a single film dosage from the unit assembly for dosing purposes. Further, the individual film strips may be desirably configured to correspond to the buccal (inner cheek) area (trapezoid or triangle), or to the sublingual area beneath the tongue of a user (e.g., trapezoid or triangle), or generally to lie along the tongue with a larger portion of the film positioned towards the front or back of the mouth (e.g., L-shape).

The unit assembly includes individual film dosages that are visually identifiable and distinguishable from one another, for example, through the use of one or more visual cues. The films may be similarly shaped for efficiency in manufacturing and to ensure consistent dosages per film strip. However, in the packaging of the unit doses, the film strips may be rotated from one another so that the film dosages do not completely overlap one another. This may be accomplished through the use of one or more shapes or designs that are not perfectly symmetrical and/or when overlaid on each other do not perfectly overlap. Such shapes may include, for example, trapezoids, L-shaped polygons, triangles, and the like, as may be desired.

The unit assembly thus includes a plurality of individual film strips, each film strip with a dosage of one or more desired agents for administration to an individual. For dosing purposes, the individual films may be configured to be recognizable and distinguishable from one another to aid a user in removing the proper dosage from the unit assembly for ingestion and administration. Thus, the unit assembly, apparatus, and methods of the present invention are advantageous in that the embodiments promote effective and efficient dosing from a multi-pack unit assembly. A major drawback of traditional multi-pack assemblies has been that the film strips of identical shape and orientation within the casing may stick together to resemble a single dosage, even to the most careful of user. Thus, double dosages or more could be accidentally ingested repeatedly by users.

The embodiments of the present invention may employ various additional cues to a user, in addition to differing perimeter profiles, to provide additional guidance to a user and to promote ease of use. In addition to using various shapes, colors, transparency/opaqueness, writing an uneven overlay of the films and shapes or lines may be imprinted, indented or embossed onto the face or surface of each individual film strip.

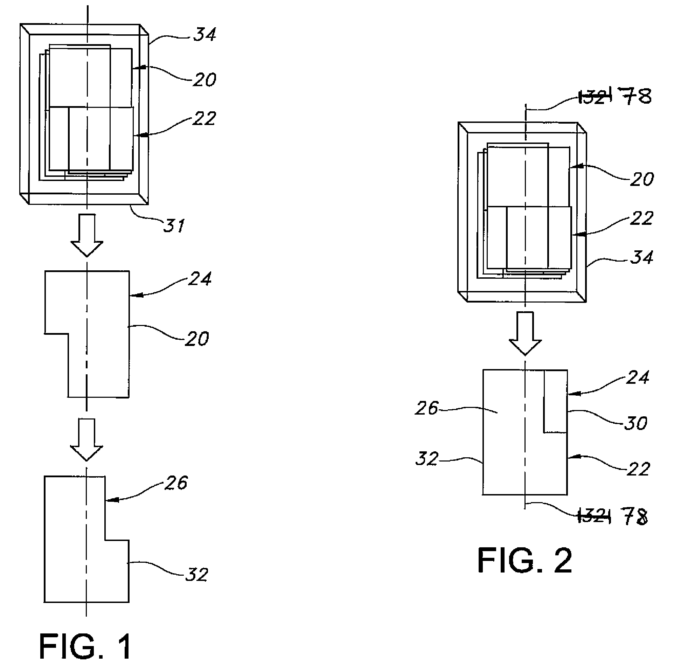

Referring to Fig. 1, an embodiment of a unit assembly 20 is provided. The unit assembly 20 includes a plurality of individual film strips 22, each of the film strips 22 having a respective perimetrical edge. The plurality of individual film strips 22 may be alternatingly positioned next to one another such that one film strip perimetrical edge 30 of a first film strip 24 is at least partially offset from a subsequent film strip perimetrical edge 32 of a second film 26. As such, the respective perimetrical edges 30, 32 of the film strips 24, 26 are directly adjacent to each other. The perimetrical edges 30, 32 of the film strips 24, 26 the surfaces of which are directly adjacent to one another are asymmetrical about a central plane 78 of the unit assembly 20. The central plane 78 is perpendicular to the directly adjacent film strips 24, 26.

In Fig. 1 and 2, the perimetrical edge of each individual of the plurality of film strips is in an L-shape. Thus, as shown in Fig. 2, if a user removes more than one film strip from the unit assembly 20, the perimeter of the film dosage appears as a rectangle, due to the overlap of film strips 22. Thus, a user would be immediately alerted that there was more than one film strip so the user would remove one or more film strips from the removed dosage. The differences in the profile of an individual film dosage compared to multiple dosages prevent the ingestion of more than one dosage by a user.

As previously noted, various other indicia, such as color, texture, design imprints or embossing may be used in combination with film shapes and juxtapositions. Such combinations apply to all embodiments discussed in this invention.

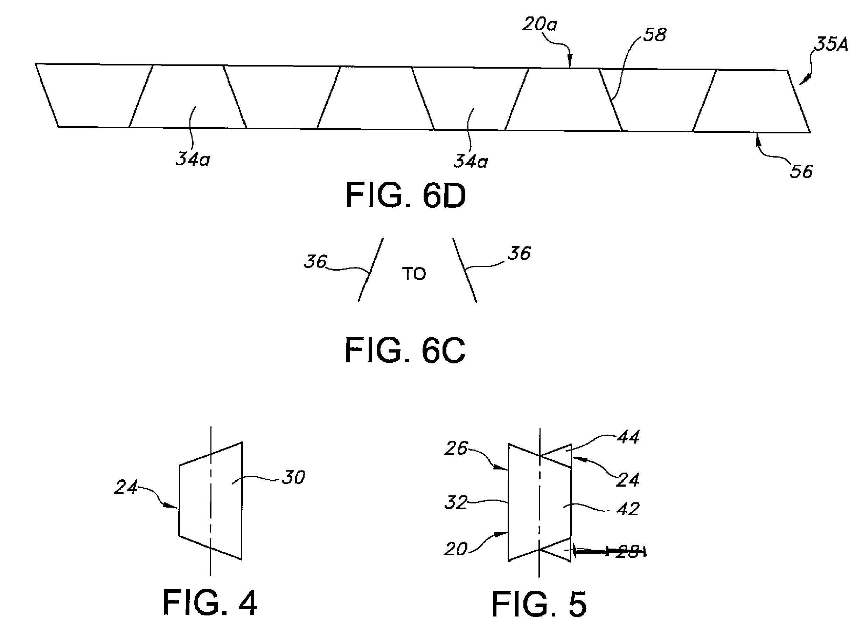

In Figs. 4 and 5, the perimeter edge of the film strips 22 is in the shape of a trapezoid, a four sided polygon. If a user removes more than one film strip from the unit assembly 20, the perimeter of the dosage or removed film strips depicts a six sided polygon, not a four sided trapezoid.

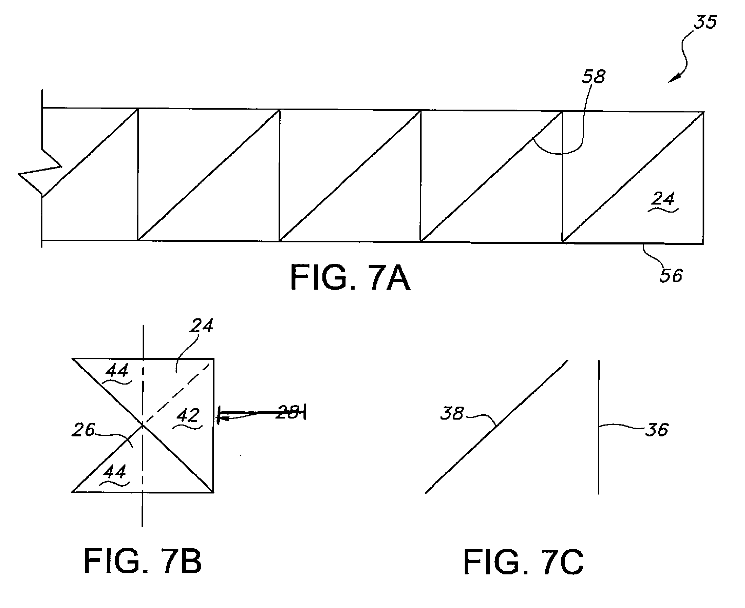

In Figs. 7A and 7B, the perimeter edge of the film strips 22 is in the shape of a triangle, specifically, a right triangle. If a user removes more than one film strip from the unit assembly 20, the perimeter of the dosage removed represents a five-sided polygon or a four-sided polygon (depending on the triangle shape employed) rather than a three-sided triangle.

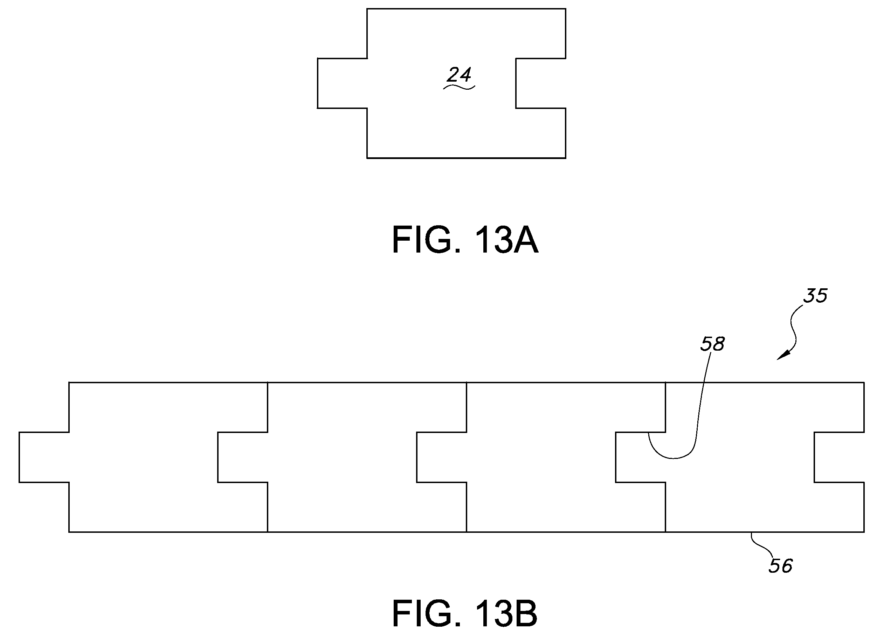

Referring to Fig. 13A and 13B, the film strips may also be in the shape of a generally rectangular, four-sided shape, but with a tab missing on one side with a tab extending from another side, the two sides opposing one another. In such a fashion, the film strips may be cut from a sheet with a blade (as shown in 13B) prior to stacking.

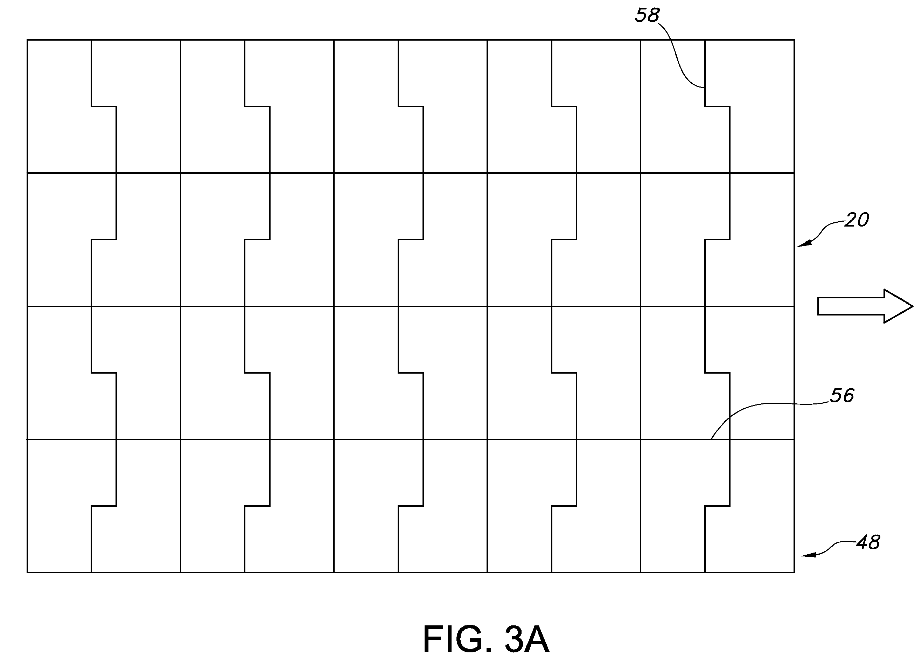

Various shapes and designs of the film strips may be employed, as may be desired. Shapes of film strips to be used with the embodiments of the present invention may include one or more desirable and/or advantageous characteristic. Specifically, it may be desirable for the film strips to be of the same shape in order to ensure that each film strip has an identical amount of active therein to correspond to a consistent dosage. It is also be desirable for the film strips to be of a shape which may be rotated and overlaid upon another film strip to create a portion of overlap, or shared perimeter and a portion of non-overlap in order to provide a shape for individual strips that is different and distinct from the profile or perimeter of a plurality of strips. One way of achieving this characteristic may be through the use of shapes that are asymmetrical, but have mirror symmetry. It may also be desirable for the film shape to use as much surface area as possible from a sheet of film in order to promote cost effectiveness and efficiency in manufacturing and prevent wasted film bi-product. It may further be desirable to use a shape that maybe repeated along the length and width of a sheet of film that has at least one common cut line, as shown in Figs. 3A, 6A, and 7A so that at least one common cutter tool may be used throughout a sheet of film in the manufacturing process, and further, one continuous cut may be made along a large sheet.

Each of the plurality of film strips 22 further may desirably include an active within a polymer matrix. The active may include a pharmaceutical, a nutraceutical, and/or other agents, as may be desired. The polymer matrix may desirably be ingestible and/or rapidly solubilizable. Controlled release, rapid release, sequential release, pulse release and sustained release compositions, among others, may be employed to give the desired release profile. Thus, the individual film strip may contain a dosage of a medicament or an agent which is to be administered and dissolved in a user's orifice, including the mouth, e.g., cheek, or under or on the tongue; mucosal membrane area such as the vagina, anus, eyes; or on a wound, or on the skin, e.g., via a transdermal application.

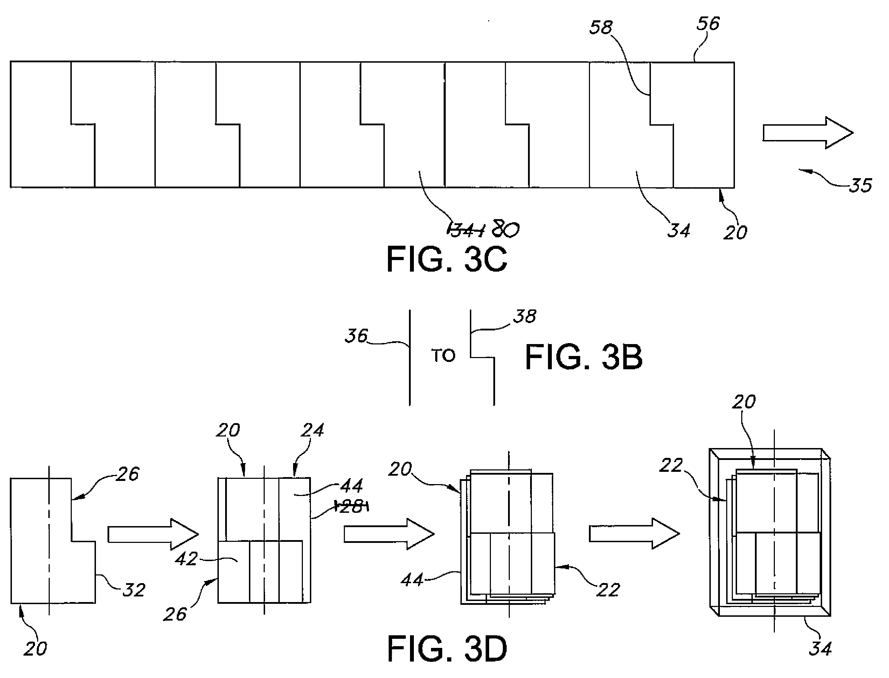

The unit assembly 20 may be stored in a cassette 34 or in an encasing member, as shown in Figs. 1 and 2. The unit assembly 20 as shown in Figs. 1 and 2 allows for multiple films 22 to be stored in a single location and withdrawn individually promoting proper dosing. In Fig. 1 and 2, the plurality of films 22 share an L-shape. Other shapes may be used, including trapezoids as shown in Figs. 4-6D or triangles as shown in Figs. 7A-7C. When an individual withdraws the film 22 from the unit assembly 20 one at a time, the individual films 24 and 26 have a separate and distinct profile or perimeter from the profile or perimeter of a plurality film strips 22. This is depicted, for example, in Fig. 3D, Fig. 4, Fig. 5, Fig. 7A and Fig. 7B.

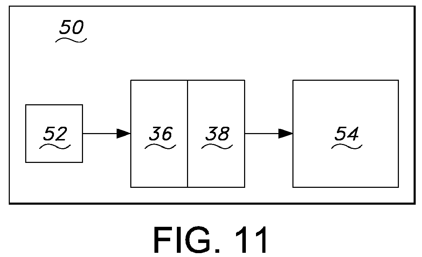

Referring to Fig. 11, a flow chart depicting an apparatus 50 for making a unit assembly 20 is provided. The apparatus 50 includes: a mandrel 52 and at least two blades 36, 38. The mandrel 52 supports the sheet of film 48. The sheet of film 48 includes a polymer matrix and an active, as in the individual film strips 22. The mandrel 52 supports the sheet 48 while it is cut by the at least two blades 36, 38. The blades 36, 38 may operate sequentially inline to cut individual film strips from the sheet. The blades may also operate alternatingly to first cut the sheet 48 in one direction with a first blade 36 followed by a second cut in another direction by a second blade 38 to yield individual film strips 22.

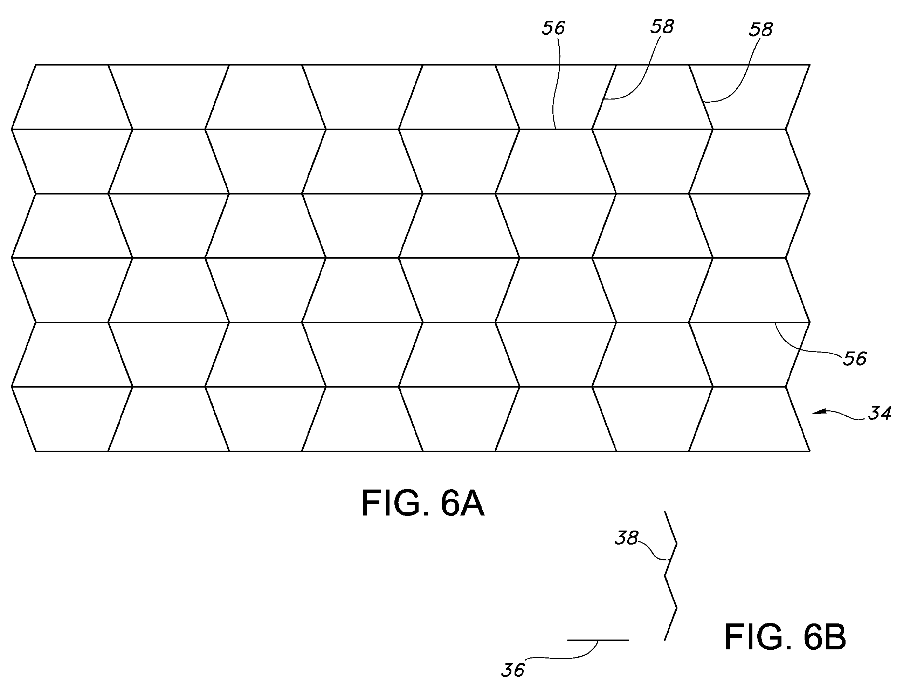

The sheet 48 may be cut into a plurality of triangle film strips, trapezoid film strips, L-shaped film strips, and the like, as shown in Fig. 3A, 6A and 7A. The first blade 36 of the apparatus 50 may have a straight cutting edge to cut the sheet 48 in a straight line. The second blade 38 may have a zigzag blade, or other shaped blade, as in Fig. 6B or a stepped chopping blade as in Fig. 3B.

At least one of the blades may be rotatable. For example, if the second blade 38 is rotatable, then a zigzag blade may be replaced with a straight, conventional cutting edge on the second blade 38. Thus, a first pass may be from the first blade to cut the sheet into long lengths, while the second pass may be from a rotatable second blade 38. The blade may rotate from about 10 degrees up to about 60 degrees. Preferably, the blade is rotatable from about 30 degrees to about 45 degrees back and forth in order to cut the smaller sheets 48 into the individual film strips. To rotate the second blade, the apparatus 50 may further include a tool 54 to hold and rotate the blade 38. The alternating inclinations of the cutting edge of the blade 38 may be provided by a mechanical or electromechanical device which reorients the blade 38 between successive cuts of the sheet 48 by the cutting edge. Alternately, the blades 36, 38 may remain in a non-rotatable position. Rather, the sheet 48 may be rotated and/or configured so that the blades 36/38 may make the appropriate cuts to reduce the sheet 48 to a plurality of individual film strips 22.

The tool 54 supports the fist and second blades 36, 38, and may be movable relative to the mandrel 52 such that, when the sheet 48 is supported on the mandrel 52, the tool 54 forces the cutting edges of the first and second blades 36, 38 through the sheet 48. The respective first and second blades 36, 38 sever alternating parts of the sheet 48 to form the plurality of individual film strips 22.

The sheet 48 may be cut in a variety of different configurations, as may be desired. One such configuration is to first cut the sheet 48 by forcing the cutting edge of the first blade 36 (straight edge) through the sheet 48 to cut a plurality of long sheets, smaller sheets from the large sheet 48. Such a cut will allow for the plurality of long, smaller sheets to continue to be supported by the mandrel and conveyed along the manufacturing line or production line. The first cut by the first blade 36 is depicted in Fig. 3A, 6A and 7A by the margins 56. Another cutting step may be completed by the second blade 38, which may have a straight blade, a straight rotatable blade, a stepped blade, or a zigzag or other shaped blade, as may be desired. The second cutting margin 58 is depicted in Figs. 3A, 6A and 7A: Additional cutting steps of the sheet 48 may precede or follow the cutting thereof by the first and second blades 36, 38. Consequently, the plurality of individual film strips 22 are formed from the sheet 48. The individual film strips 22 may be sorted into the desired configuration prior to packaging or assembly into the unit assembly 20. Such sorting may include rotating alternating film strips to a predetermined angle. For example, as with the triangle, trapezoid, and L-shaped sections strips, the strips maybe rotated to 180 degree difference. This rotating step allows for the individual film strips 22 to be overlaid and share a common overlay portion and a distinct discontinuous portion that is not overlapped, ensuring that the profile or perimeter of the individual film strip is different and distinct from the profile or perimeter of two or more film strips. Sorters and packaging tools may be included with the apparatus 50, as may be desired, in order to configure the plurality of individual film strips 22 into the unit assembly 20 with the desired configuration.

In addition, it may be desirable to include a static Free or reduced environment in the cutting or stacking of the strips 22 in the unit assembly 20. Thus, the accumulation of electrical charges, which may occur in the conveying and assembly of the strips into packages, will not interfere with the film strip placement into unit assembly cassettes, packages, and the like.

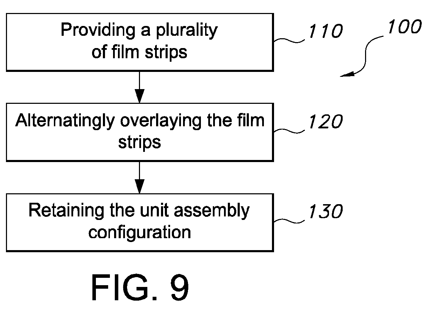

Referring to Fig. 9, a method 100 for making a unit assembly 20 is provided. The method 100 includes the steps of: providing 110 at least two individual polymeric film strips 24, 26, the film strips 24, 26 having respective perimetrical edges; and alternatingly overlaying 120 the film strips 24, 26 onto one another such that the respective perimetrical edge of a first film strip 24 is at least partially offset from the perimetrical edge of the other film strip 26 which are directly adjacent to the first film strip 24. The method 100 may include the alternating overlaid arrangement of a plurality of film strips 22 in order to configure and complete a unit assembly 20. The method 100 may further include the step of retaining 130 the unit assembly 20. The retaining 130 step may include packaging or retaining the unit assembly 20 within a cassette 34 or an encasing member. Such a cassette 34 may prevent the unit assembly 20 from interaction with contaminants and protect the structural integrity of the film strips 22 between administration times of a user. The cassette 34 may be composed or one or more materials, as may be desired.

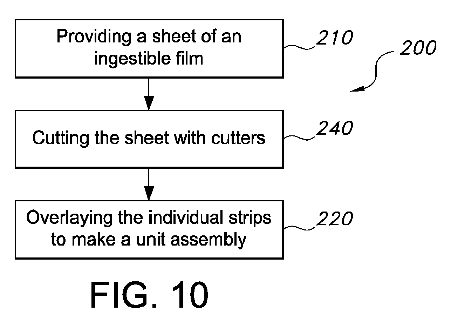

Referring to Fig. 10, another method 200 for making a unit assembly 20 is provided. The method 200 includes the steps of: providing 210 a sheet 48 of an ingestible film, the film including a polymeric matrix having an active therein; cutting 240 the sheet 48 with at least a first cutter 36 and a second cutter 38, the first cutter 36 having a first orientation generally parallel to a longitudinal axis of the sheet 48, the second cutter 38 having a second orientation generally perpendicular to the longitudinal axis of the sheet 48, wherein the first and second cutters 36, 38 create a plurality of individual film strips 22 having substantially the same shape and size from the sheet; overlaying 220 the individual film strips 22 into a unit assembly 20 such that each film strip 22 does not completely overlay the perimeter of its adjacent film strips 22.

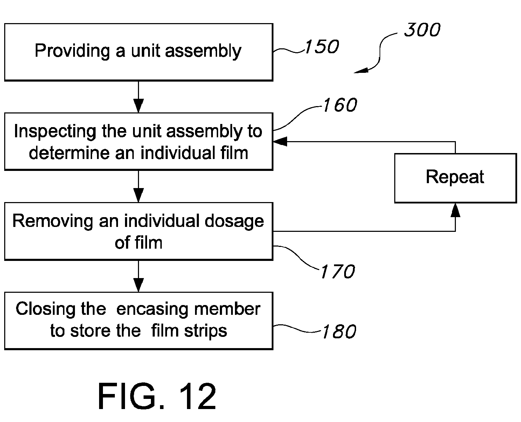

Referring to Fig. 12, a method 300 of dosage retrieval is provided. The method 300 includes the steps of: providing 150 a unit assembly 20 including a plurality of individual film strips 22 packaged therein, each film strip optionally having an active within a polymer matrix; inspecting 160 the unit assembly 20 for a cue 58 to determine a dosage to be removed therefrom; and removing 170 a dosage from the unit assembly 20. As previously discussed, the unit assembly 20 may include a cassette 34 to enclose and protect and/or store the plurality of film strips 22. Thus, the method 300 may further include the step of closing the encasing member or cassette 34 to protect and/or store the film strips 22 within and optionally comparing an instruction on said encasing to said individual film strip removed from said unit assembly. The method 300 may be repeated, as a user may need to administer dosages of film strips 22. A dosage as used herein, is preferably an individual film strip 24, to be administered to a user in need of the agent or medicament therein.

In an alternative embodiment, the sheet 48 is cut into the ribbons 35 without the formation of the L-shaped sections 80 in the sheet. In this embodiment, the respective blades 36, 38 sever alternating parts of the ribbons 35 to form the L-shaped sections 80, as shown in Fig. 3C. The L-shaped sections 80 are separated from the ribbons 35 to form the film strips 24, 26.

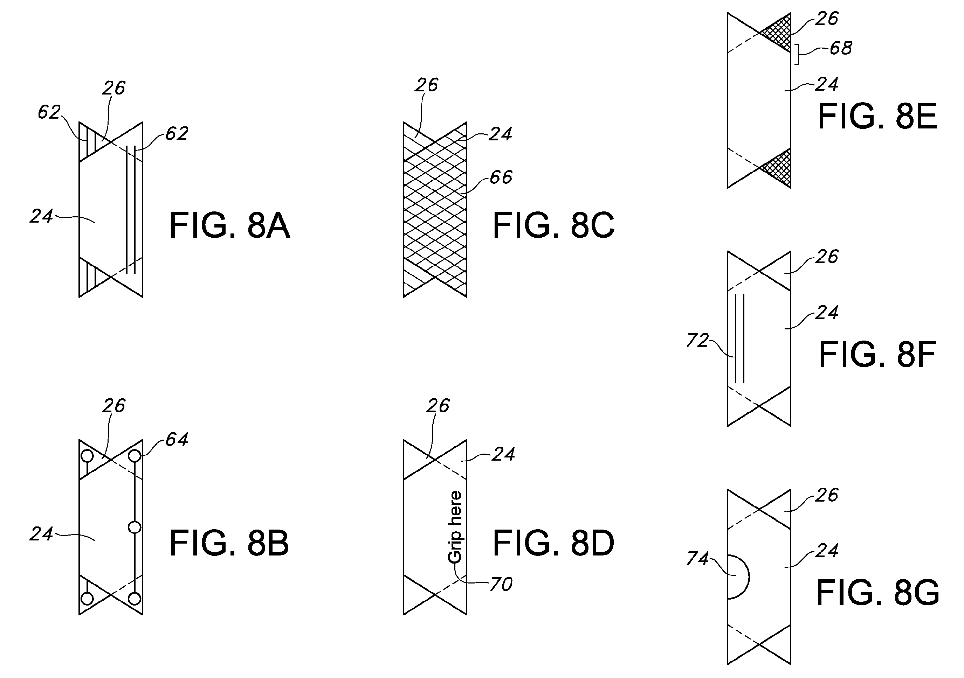

Referring to Figs. 8A through 8G, additional visual cues 70 of the unit assembly 20 is provided. Though trapezoid-shaped film strips were employed through this section of examples and related Fig. 8A through 8G, it should be understood that various other shapes and configurations may be likewise employed as may be desired. Referring to Fig. 8A, an edge of each film strip 24, 26 may have at least one line 62 (as shown, two lines) across the side to indicate that film strip 24 is at the top of the unit assembly 20, to be dispensed as a dosage. Referring to Fig. 8B, the filmstrips 22 may each have a stamped cutout 64 to distinguish a single dosage from a plurality of strips 22. Referring to Fig. 8C, the film strips 22 may be a color contrast 66 of respective colors for 24 and 26, where 24, 26 are non-opaque (at least partially transparent or translucent), where each film strip (e.g., 24,26) has a respective color. When the film strips 24-26 are positioned next to one another, the respective colors of the film strips 22 that are directly adjacent to one another are different. Consequently, the visible color of the unit assembly 20 portions is different. The continuous overlap region 42 is one color based on the contrast of 24, 26, while the discontinuous overlap region 44 is the respective color of each film strip 24, 26 of the plurality of film strips.

Referring to Fig. 8E, the film strips 22 in the alternating arrangement may be of an alternating opaque color. Thus, as shown in Fig. 8E, a first film 24 has a color which is contrasted to the color of a second film strip 26. A user would thus only remove a dosage corresponding to a profile or perimeter having only 1 color to it.

Referring to Fig. 8D, each of the film strips 24, 26 may include a writing 76 thereon along a portion of the perimeter of each strip what is consistent with the continuous overlap region. The writing may refer to one or more indices, as may be desired. The film strips are positioned to overlay one another such that only the indicia of the top-most film strip are detachable.

Referring to Fig. 8F, the filmstrips 22 may include an indentation 72 as a visual cue 70. Thus, the indentation 72 may also serve as a tactile cue, which may be readily detected by a user's fingertip(s). Further, the indentation 72 should be along a side of the perimetrical edge 28, which is along the continuous overlap region. As shown in Fig. 8F, only the top-most film strip, 24, is depicted as having the indentation along the full length of its side.

Referring to Fig. 8G, the visual cue 70 may further take the form of a fill 74, which may be laid upon the face of each film strip, such that only one fill 74 is showing on the top-most film strip 24 of the unit assembly. The fill portion 74 may include a flavorful, edible, not overly hydrophilic base. Thus, when a user places a dosage in their mouth, the fill may counter the taste of the film, including the agents thereon, with a pleasant after taste. Thus, the visual cue 70 in the form of a fill may allow a user to distinguish and remove a single dosage from the unit assembly 20, and may also promote a desired taste sensation to a user upon administration.

## Claims

1. A unit assembly comprising:
a plurality of individual film strips, each film strip having an active within a polymer matrix, said film strips having respective perimetrical edges,
said plurality of individual film strips being alternatingly positioned next to one another such that a film strip perimetrical edge is at least partially offset from a subsequent film strip perimetrical edge, wherein said respective perimetrical edges of said film strips are directly adjacent to each other.

2. The unit assembly of claim 1, further wherein said perimetrical edges of said film strips which are directly adjacent to one another are symmetrical about a central plane of said unit assembly which is perpendicular to said directly adjacent film strips.

3. The unit assembly of claim 1, further wherein said perimetrical edges define respective geometric shapes.

4. The unit assembly of claim 1, further comprising a visual indicia for indicating a single dosage of the unit assembly.

5. The unit assembly of claim 1, further comprising an encasing member to encase the plurality of film strips.

6. The unit assembly of claim 1, further wherein the plurality of film strips are oriented at a different angle from one another, so that a film strip and a subsequent film strip, though of substantially similar size and shape, are easily distinguishable from one another as the film strip and the subsequent film strip together have a discontinuous overlay region.

7. A method for making a unit assembly, said method comprising:
providing at least two individual polymeric film strips, the film strips having respective perimetrical edges; and
alternatingly overlaying the film strips onto one another such that the respective perimetrical edges of a first film strip is at least partially offset from the perimetrical edges of the each of the other film strips which are directly adjacent to a first film strip.

8. The method of claim 7, further wherein the film strips are in a geometrical shape selected from one of the following, including: a trapezoid, a parallelogram, a triangle, a right triangle, an L-shape, an tabbed-octagon, an asymmetrical shape, and combinations thereof.

9. A method for making a unit assembly, said method comprising:
providing a sheet of an ingestible film, the film including a polymeric matrix having an active therein;
cutting the sheet with at least a first cutter and a second cutter, said first cutter having a first orientation generally parallel to a longitudinal axis of said sheet, said second cutter having a second orientation generally perpendicular to said longitudinal axis of said sheet, wherein the first and second cutters create a plurality of individual film strips having substantially the same shape and size from said sheet; and
overlaying the individual film strips into a unit assembly such that each film strip does not completely overlay the perimeter of its adjacent film strips.

10. The method of claim 9, wherein the overlaying step further comprises one of the following steps:
(i) stacking the individual film strips when the cutting step yields individual film strips having the substantially the same shape, but in different orientations to adjacent individual film strips; or
(ii) rotating the film strips in alternating fashion when the cutting step yields individual film strips having substantially the same shape but in the same orientations as adjacent individual film strips.

11. The method of claim 9, wherein the cutting step further comprises said first cutter with a straight blade and said second cutter with a stepped blade or zigzagged blade.

12. An apparatus for making a unit assembly as defined in claim 1 comprising:
a mandrel on which a sheet of film is supported;
at least two blades, a first blade having a first position relative to a sheet position and a second blade having an adjustable position relative to the sheet position wherein said second blade is rotatable.

13. A method of dosage retrieval, comprising:
providing a unit assembly as defined in claim 1 including a plurality of individual film strips packaged therein, each film strip having an active within a polymer matrix;
inspecting the individual film strip for a cue that the individual film is a single dosage; and
removing a individual film strip from said unit assembly.

14. The method of claim 13, further comprising closing an encasing member, for encasing the film strips between the strip removal.

15. The method of claim 13, further comprising one of the following steps:
(i) comparing the shape of the individual film strip to the shape of the unit assembly;
(ii) comparing an individual film strip color to a unit assembly color; or
(iii) comparing an individual film strip indicia to a unit assembly indicia.

## Patentansprüche

1. Verbundeinheit umfassend:
mehrere einzelne Filmstreifen, die jeweils einen Wirkstoff in einer Polymermatrix aufweisen, wobei jeder Filmstreifen Randkanten hat,
wobei die mehreren einzelnen Filmstreifen derart im Wechsel nebeneinander angeordnet sind, dass eine Randkante eines Filmstreifens zumindest teilweise im Versatz von einer Randkante eines darauffolgenden Filmstreifens angeordnet ist, wobei die jeweiligen Randkanten der Filmstreifen unmittelbar benachbart sind.

2. Verbundeinheit nach Anspruch 1, wobei ferner die Randkanten unmittelbar benachbarter Filmstreifen symmetrisch bezüglich einer zentralen Ebene der Verbundeinheit sind, die senkrecht zu den unmittelbar benachbarten Filmstreifen ist.

3. Verbundeinheit nach Anspruch 1, wobei ferner die Randkanten jeweilige geometrische Formen definieren.

4. Verbundeinheit nach Anspruch 1, ferner umfassend ein visuell wahrnehmbares Erkennungszeichen zum Erkennen einer Einzeldosis der Verbundeinheit.

5. Verbundeinheit nach Anspruch 1, ferner umfasend ein Behälterelement zum Umhüllen der mehreren Filmstreifen.

6. Verbundeinheit nach Anspruch 1, wobei ferner die mehreren Filmstreifen in verschiedenen Winkeln zueinander orientiert sind, so dass ein Filmstreifen und ein darauffolgender Filmstreifen, obwohl sie im Wesentlichen gleiche Größe und gleiche Form aufweisen, leicht voneinander unterscheidbar sind, da der Filmstreifen und der darauffolgende Filmstreifen zusammen einen diskontinuierlichen Überlappungsbereich aufweisen.

7. Verfahren zur Herstellung einer Verbundeinheit, wobei das Verfahren umfasst:
Bereitstellen von mindestens zwei einzelnen Polymerfilmstreifen, die jeweilige Randkanten haben; und
Alternierendes Übereinanderlegen der Filmstreifen derart, dass die jeweiligen Randkanten eines ersten Filmstreifens mindestens teilweise im Versatz zu den Randkanten jedes der anderen Filmstreifen, die dem ersten Filmstreifen direkt benachbart sind, angeordnet sind.

8. Verfahren nach Anspruch 7, wobei ferner die Filmstreifen eine geometrische Form haben, die aus einer der folgenden ausgewählt ist: Trapezoid, Parallelogramm, Dreieck, rechtwinkliges Dreieck, L-Form, Achteck mit Laschen, asymmetrische Form und Kombinationen davon.

9. Verfahren zur Herstellung einer Verbundeinheit, wobei das Verfahren umfasst:
Bereitstellung einer Bahn eines ingestiblen Films, wobei der Film eine Polymermatrix mit einem darin enthaltenen Wirkstoff einschliesst;
Zuschneiden der Bahn mit mindestens einer ersten Schneideeinrichtung und einer zweiten Schneideeinrichtung, wobei die erste Schneideeinrichtung eine erste Orientierung hat, die allgemein parallel zu einer Längsachse der Bahn ist, und die zweite Schneideeinrichtung eine zweite Orientierung hat, die allgemein senkrecht zur Längsachse der Bahn ist, wobei die erste und zweite Schneideeinrichtung aus der Bahn mehrere einzelne Filmstreifen erzeugen, die im Wesentlichen die gleiche Form und die gleiche Größe aufweisen; und
Übereinanderlegen der einzelnen Filmstreifen zu einer Verbundeinheit derart, dass jeder Filmstreifen sich nicht vollständig mit dem Umfangsrand seiner benachbarten Filmstreifen deckt.

10. Verfahren nach Anspruch 9, wobei der Schritt des Übereinanderlegens ferner einen der folgenden Schritte umfasst:
(i) Stapeln der einzelnen Filmstreifen, wenn der Schneideschritt einzelne Filmstreifen erzeugt, die im Wesentlichen die gleiche Form, aber eine andere Orientierung als die jeweils benachbarten einzelnen Filmstreifen haben; oder
(ii) Drehen der Filmstreifen in abwechselnder Weise, wenn der Schneideschritt einzelne Filmstreifen erzeugt, die im Wesentlichen die gleiche Form und auch die gleiche Orientierung wie benachbarte einzelne Filmstreifen haben.

11. Verfahren nach Anspruch 9, wobei der Schneideschritt ferner die erste Schneideeinrichtung mit einem geraden Blatt und die zweite Schneideeinrichtung mit einem gestuften Blatt oder einem zickzackförmigen Blatt umfasst.

12. Vorrichtung zur Herstellung einer Verbundeinheit wie in Anspruch 1 definiert, wobei die Vorrichtung umfasst:
einen Dorn, auf welchem eine Filmbahn getragen wird;
mindestens zwei Blätter, wobei ein erstes Blatt eine erste Position bezüglich einer Bahnposition hat und wobei ein zweites Blatt eine anpassbare Position bezüglich der Bahnposition hat und wobei das zweite Blatt drehbar ist.

13. Verfahren zur Dosierung umfassed die Schritten:
Bereitstellen einer Verbundeinheit wie in Anspruch 1 definiert, die mehreren darin verpackten einzelnen Filmstreifen einschliesst, wobei jeder Filmstreifen einen Wirkstoff in einer Polymermatrix aufweist;
Prüfen des einzelnen Filmstreifens auf einen Hinweis, dass der einzelne Film eine Einzeldosis ist; und
Entnehmen eines einzelnen Filmstreifens von der Verbundeinheit.

14. Verfahren nach Anspruch 13, ferner umfassend Schließen eines Behälterelements, um die Filmstreifen zwischen den Filmstreifen-Entnahmen zu umhüllen.

15. Verfahren nach Anspruch 13, ferner umfassend eins der folgenden Schritte:
(i) Vergleichen der Form des einzelnen Filmstreifens mit der Form der Verbundeinheit;
(ii) Vergleichen einer Farbe eines einzelnen Filmstreifens mit einer Farbe der Verbundeinheit; oder
(iii) Vergleichen eines Erkennungszeichens eines einzelnen Filmstreifens mit einem Erkennungszeichen der Verbundeinheit.

## Revendications

1. Ensemble unitaire comprenant :
une pluralité de bandes de film individuelles, chaque bande de film ayant un actif au sein d'une matrice polymère, lesdites bandes de film ayant des bords périmétriques respectifs,
ladite pluralité de bandes de film individuelles étant placées alternativement l'une à côté de l'autre de telle sorte qu'un bord périmétrique de bande de film est au moins partiellement décalé par rapport au bord périmétrique de la bande de film suivante,
dans lequel lesdits bords périmétriques respectifs desdites bandes de film sont directement adjacents les uns aux autres.

2. Ensemble unitaire selon la revendication 1, dans lequel en outre lesdits bords périmétriques desdites bandes de film qui sont directement adjacents les uns aux autres sont symétriques par rapport à un plan central dudit ensemble unitaire qui est perpendiculaire auxdites bandes de film directement adjacentes.

3. Ensemble unitaire selon la revendication 1, dans lequel en outre lesdits bords périmétriques définissent des formes géométriques respectives.

4. Ensemble unitaire selon la revendication 1, comprenant en outre des signes visuels pour indiquer une dose unique de l'ensemble unitaire.

5. Ensemble unitaire selon la revendication 1, comprenant en outre un boîtier pour enfermer la pluralité de bandes de film.

6. Ensemble unitaire selon la revendication 1, dans lequel en outre la pluralité de bandes de film sont orientées selon un angle différent les unes des autres, de sorte que une bande de film et une bande de film suivante, bien que de taille et de forme sensiblement similaire, se distinguent facilement l'une de l'autre puisque la bande de film et la bande de film suivante ont une région de recouvrement discontinue.

7. Procédé de fabrication d'un ensemble unitaire, ledit procédé comprenant :
la fourniture d'au moins deux bandes individuelles de film polymère, les bandes de film ayant des bords périmétriques respectifs; et
la superposition alternative des bandes de film l'une sur l'autre de telle sorte que les bords périmétriques respectifs d'une première bande de film sont au moins partiellement décalés par rapport aux bords périmétriques de chacune des autres bandes de film qui sont directement adjacentes à la première bande de film.

8. Procédé selon la revendication 7, dans lequel en outre les bandes de film ont une forme géométrique choisie parmi l'une des suivantes, comprenant: un trapézoïde, un parallélogramme, un triangle, un triangle rectangle, une forme en L, un octogone à onglets, une forme asymétrique et les combinaisons de celles-ci.

9. Procédé de fabrication d'un ensemble unitaire, ledit procédé comprenant :
la fourniture d'une feuille de film comestible, le film comprenant une matrice polymère ayant un actif à l'intérieur;
la coupe de la feuille avec au moins un premier dispositif de coupe et un second dispositif de coupe, ledit premier dispositif de coupe ayant une première orientation généralement parallèle à un axe longitudinal de ladite feuille, ledit second dispositif de coupe ayant une deuxième orientation globalement perpendiculaire audit axe longitudinal de ladite feuille, dans lequel le premier et le second dispositifs de coupe créent une pluralité de bandes de film individuelles ayant sensiblement la même forme et taille à partir de ladite feuille; et
la superposition des bandes de film individuelles dans un ensemble unitaire de telle sorte que chaque bande de film ne recouvre pas complètement le périmètre de ses bandes de film adjacentes.

10. Procédé selon la revendication 9, dans lequel l'étape de superposition comprend en outre l'une des étapes suivantes :
(i) l'empilage des bandes de film individuelles lorsque à l'étape de coupe on obtient des bandes de film individuelles ayant sensiblement la même forme, mais dans des orientations différentes de celle des bandes de film adjacentes ; ou
(ii) la rotation des bandes de film de façon alternée lorsque l'étape de coupe donne des bandes de film individuelles ayant sensiblement la même forme, mais dans les mêmes orientations que celle des bandes de film individuelles adjacentes.

11. Procédé selon la revendication 9, dans lequel l'étape de coupe comprend en outre ledit premier dispositif de coupe avec une lame droite et ledit second dispositif de coupe avec une lame crantée ou en dents de scie.

12. Appareil pour fabriquer un ensemble unitaire tel que défini à la revendication 1, comprenant :
un mandrin sur lequel une feuille de film est supporté;
au moins deux lames, une première lame ayant une première position par rapport à une position de feuille et une seconde lame ayant une position réglable par rapport à la position de la feuille dans laquelle ladite seconde lame est rotative.

13. Procédé de récupération d'une dose, comprenant :
la fourniture d'un ensemble unitaire tel que défini à la revendication 1 y compris une pluralité de bandes de film individuelles contenues dans celui-ci, chaque bande de film ayant un actif au sein d'une matrice polymère;
l'inspection de la bande de film individuelle pour obtenir un indice que le film individuel est une dose unique; et
le retrait d'une bande de film individuelle dudit ensemble unitaire.

14. Procédé selon la revendication 13, comprenant en outre la fermeture d'un boîtier, pour enfermer les bandes de film pendant le retrait de la bande.

15. Procédé selon la revendication 13, comprenant en outre une des étapes suivantes :
(i) la comparaison de la forme de la bande de film individuelle avec la forme de l'ensemble unitaire ;
(ii) la comparaison de la couleur d'une bande de film individuelle avec la couleur de l'ensemble unitaire ; ou
(iii) la comparaison d'indices d'une bande de film individuelle avec des indices de l'ensemble unitaire.
